# EUROPEAN PATENT APPLICATION

(11) **EP 2 745 762 A1**
(43) Date of publication of application: **25.06.2014**
(21) Application number: 12824603.0
(22) Date of filing: 26.06.2012
(51) Int. Cl.: A61B 1/00

(54) **OPTICAL MEASURING DEVICE AND PROBE**

(30) Priority: 17.08.2011 US 201161524540 P
(71) Applicant: OLYMPUS MEDICAL SYSTEMS CORP., Tokyo 151-0072 (JP)
(72) Inventor: ITO, Seigo, Tokyo 151-0072 (JP); KOBAYASHI, Yoshimine, Tokyo 151-0072 (JP)
(74) Representative: Schicker, Silvia
(86) International application number: PCT/JP2012/066261
(87) International publication number: WO 2013/024631

(57) **Abstract**

An optical measurement apparatus 1 is an optical measurement apparatus that performs spectrometry on returned light reflected or scattered by a biological tissue 6 and obtains a characteristic value of the biological tissue 6, and includes: a probe 3 having an irradiation fiber 5 that propagates light supplied from a proximal end thereof and emits the light from a distal end thereof, and a plurality of light-receiving fibers 7 and 8, each of which propagates light entering from a distal end thereof and outputs the light from a proximal end thereof; and a balloon provided at a distal end 33 of the probe 3 to light-shield a region including a cross section of the probe 3, the cross section being perpendicular to a long axis of the probe 3, the region having an area larger than that of the cross section.

## Description

### Field

The present invention relates to an optical measurement apparatus that obtains a characteristic value of a biological tissue by performing spectrometry on returned light reflected or scattered by the biological tissue and relates to a probe having an irradiation fiber that propagates light supplied from a proximal end thereof and emits the light from a distal end thereof and a plurality of light-receiving fibers, each of which propagates light entering from a distal end thereof and outputs the light from a proximal end thereof.

### Background

In recent years, an optical measurement apparatus has been proposed, which uses a low-coherence enhanced backscattering (LEBS) technique for detecting characteristics of a biological tissue by irradiating the biological tissue, which is a scatterer, with low-coherent light having a short spatial coherence length from a distal end of a probe and measuring an intensity distribution of scattered light (for example, see Patent Literature 1 and 2). Such an optical measurement apparatus performs optical measurement on an object to be measured, such as a biological tissue, in combination with an endoscope for observing internal organs such as digestive organs.

The optical measurement apparatus using this LEBS technique, by obtaining scattered light beams having a plurality of desired angles using a plurality of light-receiving fibers and thereafter performing spectrometry with a measurement device, obtains the intensity distribution of the scattered light from the biological tissue and obtains a characteristic value related to characteristics of the biological tissue based on results of this measurement.

### Citation List

### Patent Literature

Patent Literature 1: International Patent Publication Pamphlet No. WO2007/133684
Patent Literature 2: U.S. Patent Application Laid-open No. 2008/0037024

### Summary

### Technical Problem

In the optical measurement apparatus disclosed in Literature 1 and 2, since white light similar to endoscopic illumination light is used for the measurement, the endoscopic illumination light that has entered inside a probe from a lateral side of a distal end of the probe or the endoscopic illumination light that has reached a light-receiving fiber via a surface layer of the biological tissue around the distal end of the probe becomes noise.
In particular, red light having a long wavelength from components included in endoscopic illumination light is known to easily reach light-receiving fibers and to often exert influence as noise. When there is much of such noise, there are problems that optical properties of the biological tissue are not accurately obtainable, and detection accuracy of characteristics of the biological tissue is reduced.

The present invention has been made in view of the above, and its object is to provide an optical measurement apparatus and a probe that are able to obtain a measurement value having little noise caused by endoscopic illumination light.

### Solution to Problem

To solve the above problem and achieve the object, an optical measurement apparatus according to the present invention is an optical measurement apparatus that performs spectrometry on returned light reflected or scattered by a biological tissue and obtains a characteristic value of the biological tissue, and the optical measurement apparatus includes: a probe having: an irradiation fiber that propagates light supplied from a proximal end thereof and emits the light from a distal end thereof; and a plurality of light-receiving fibers, each of which propagates light entering from a distal end thereof and outputs the light from a proximal end thereof; and a light-shielding member provided at a distal end of the probe to light-shield a region including a cross section of the probe, the cross section being perpendicular to a long axis of the probe, the region having an area larger than that of the cross section.

Further, the optical measurement apparatus according to the present invention is characterized in that the light-shielding member is a balloon that inflates in a radial direction of the probe, the probe has a fluid supply tube of which a distal end communicates with inside of the balloon and a proximal end extends from a proximal end portion of the probe, and the optical measurement apparatus further comprises a fluid supply unit that supplies a fluid to the fluid supply tube.

Further, the optical measurement apparatus according to the present invention is characterized in that it includes: a light source that generates light to emit the biological tissue and supplies the light to the irradiation fiber; a measurement unit that performs spectrometry on the returned light from the biological tissue output by each of the plurality of the light-receiving fibers; a detection unit that detects a state of inflation of the balloon; and a control unit that causes a light emission process by the light source and a spectrometry process by the measurement unit to be startable when inflation of the balloon is detected by the detection unit.

Further, the optical measurement apparatus according to the present invention is characterized in that the light-shielding member is a light-shielding plate that is foldable along an outer side face of the probe.

Further, the optical measurement apparatus according to the present invention is characterized in that the light-shielding member is a hood that deforms into a shape protruding towards a radial direction of the probe when the distal end of the probe is pushed against the biological tissue.

Further, an optical measurement apparatus according to the present invention is an optical measurement apparatus including a probe having an irradiation fiber that propagates light supplied from a proximal end thereof and emits the light from a distal end thereof and a plurality of light-receiving fibers, each of which propagates light entering from a distal end thereof and outputs the light from a proximal end thereof, the optical measurement apparatus performing spectrometry on returned light reflected or scattered by a biological tissue and obtains a characteristic value of the biological tissue, wherein the optical measurement apparatus further includes: a draw-in unit that causes ambient light coming from outside of the probe to attenuate within the biological tissue before the ambient light reaches the light-receiving fibers by drawing the biological tissue making contact with the distal end of the probe into a space for draw-in provided in the probe.

Further, the optical measurement apparatus according to the present invention is characterized in that the draw-in unit is a suction pressure supply unit that supplies a suction pressure to the space.

Further, the optical measurement apparatus according to the present invention is characterized in that the draw-in unit is a gripping unit that grips a part of the biological tissue and pulls the part of the biological tissue into the space.

Further, a probe according to the present invention is a probe having: an irradiation fiber that propagates light supplied from a proximal end thereof and emits the light from a distal end thereof; and a plurality of light-receiving fibers, each of which propagates light entering from a distal end thereof and outputs the light from a proximal end thereof; wherein the probe includes: a light-shielding member provided at a distal end of the probe to light-shield a region including a cross section of the probe, the cross section being perpendicular to a long axis of the probe, the region having an area larger than that of the cross section.

Further, the probe according to the present invention is characterized in that the light-shielding member is a balloon that inflates in a radial direction of the probe, and the probe further comprises a fluid supply tube, of which a distal end communicates with inside of the balloon and a proximal end extends from a proximal end portion of the probe.

Further, the probe according to the present invention is characterized in that the light-shielding member is a light-shielding plate that is foldable along an outer side face of the probe.

Further, the probe according to the present invention is characterized in that the light-shielding member is a hood that deforms into a shape protruding towards a radial direction of the probe when the distal end of the probe is pushed against the biological tissue.

### Advantageous Effects of Invention

In an optical measurement apparatus according to the present invention; by providing a distal end of a probe with a light-shielding member that light-shields an area larger than an area of a cross sectional shape of the probe on a plane perpendicular to a long axis of the probe to light-shield endoscopic illumination light with a lateral side of the distal end of the probe, and even when the endoscopic illumination light has reached a biological tissue, by increasing a distance between the distal end of the probe and a position at which the endoscopic illumination light reaches the biological tissue to attenuate the endoscopic illumination light inside the biological tissue; it is possible to obtain a measurement value having little noise caused by the endoscopic illumination light.

In the optical measurement apparatus according to the present invention, a distance over which the endoscopic illumination light passes through the biological tissue is increased to attenuate the endoscopic illumination light inside the biological tissue by pulling the biological tissue in contact with the distal end of the probe into a space in the distal end of the probe, and thus it is possible to obtain a measurement value having little noise caused by the endoscopic illumination light.

In a probe according to the present invention; by providing a distal end of a probe with a light-shielding member that light-shields an area larger than that of a cross-sectional shape of the probe in a plane perpendicular to a long axis of the probe to light-shield endoscopic illumination light with a lateral side of the distal end of the probe, and even when the endoscopic illumination light has reached a biological tissue, by increasing a distance between the distal end of the probe and a position at which the endoscopic illumination light reaches the biological tissue to attenuate the endoscopic illumination light inside the biological tissue; it is possible prevent the endoscopic illumination light from reaching a light-receiving fiber.

### Brief Description of Drawings

Fig. 1 is a schematic diagram illustrating a schematic configuration of an optical measurement apparatus according to a first embodiment.
Fig. 2 is a diagram illustrating a configuration of an endoscope system and a mode of installing a probe in the optical measurement apparatus.
Fig. 3 is a schematic diagram illustrating a state in which the probe of Fig. 1 has been inserted into an insertion portion of an endoscope.
Fig. 4 is a cross-sectional view illustrating a distal end portion of the probe of Fig. 3 cut along a central axis in a longitudinal direction of the probe.
Fig. 5 is a schematic diagram illustrating a state of the probe of Fig. 1 upon measurement.
Fig. 6 is a cross-sectional view illustrating the distal end portion of the probe of Fig. 5 cut along a central axis in the longitudinal direction of the probe.
Fig. 7 is a schematic diagram illustrating a schematic configuration of an optical measurement apparatus according to a second embodiment.
Fig. 8 is a flowchart illustrating an optical measurement processing sequence of the optical measurement apparatus of Fig. 7.
Fig. 9 is a schematic diagram illustrating a schematic configuration of an optical measurement apparatus according to a third embodiment.
Fig. 10 is a perspective view illustrating a distal end portion of a probe of Fig. 9.
Fig. 11 is a view taken in a direction of an arrow A of Fig. 10.
Fig. 12 is a diagram illustrating insertion of the probe into the endoscope of Fig. 9.
Fig. 13 is a diagram illustrating pull-out of the probe from the endoscope of Fig. 9.
Fig. 14 is a cross-sectional view illustrating the distal end portion of the probe upon measurement of Fig. 9 cut along a central axis of a longitudinal direction of the probe.
Fig. 15 is a schematic diagram illustrating a schematic configuration of an optical measurement apparatus according to a fourth embodiment.
Fig. 16A is a cross-sectional view illustrating a distal end portion of a probe of Fig. 15 cut along a central axis of a longitudinal direction of the probe.
Fig. 16B is a cross-sectional view illustrating the distal end portion of the probe of Fig. 15 cut along the central axis of the longitudinal direction of the probe.
Fig. 17 is a schematic diagram illustrating a schematic configuration of an optical measurement apparatus according to a fifth embodiment.
Fig. 18A is a cross-sectional view illustrating a distal end portion of a probe of Fig. 17 cut along a central axis of a longitudinal direction of the probe.
Fig. 18B is a cross-sectional view illustrating the distal end portion of the probe of Fig. 17 cut along the central axis of the longitudinal direction of the probe.
Fig. 19 is a schematic diagram illustrating a schematic configuration of an optical measurement apparatus according to a sixth embodiment.
Fig. 20A is a cross-sectional view illustrating a distal end portion of a probe of Fig. 19 cut along a central axis of a longitudinal direction of the probe.
Fig. 20B is a cross-sectional view illustrating the distal end portion of the probe of Fig. 19 cut along the central axis of the longitudinal direction of the probe.
Fig. 21 is a cross-sectional view taken along a line A-A of Fig. 20B.
Fig. 22 is another example of the cross-sectional view taken along the line A-A of Fig. 20B.

### Description of Embodiments

Hereinafter, an optical measurement apparatus using the LEBS technique according to an exemplary embodiment of the invention will be described in detail with reference to the accompanying drawings. The present invention is not limited by these embodiments. In the description of drawings, like reference numerals denote like elements. Further, it is to be noted that the drawings are schematic, and relations between thicknesses and widths of each element, and ratios among elements are different from those of the actual. Among the drawings also, a same portion having relations or ratios of dimensions different from one another is included.

### (First Embodiment)

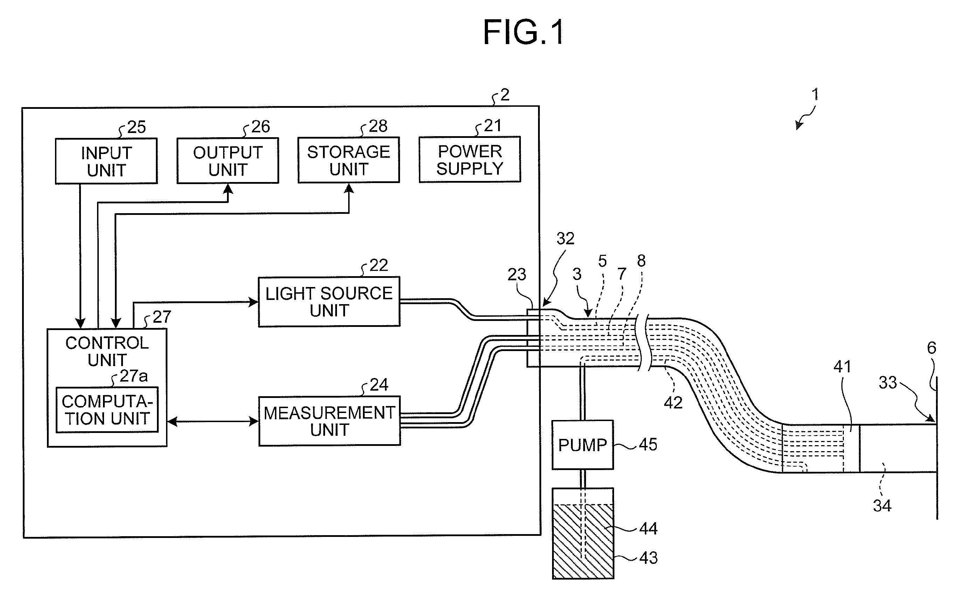

Fig. 1 is a schematic diagram illustrating a schematic configuration of an optical measurement apparatus according to a first embodiment of the invention. As illustrated in Fig. 1, an optical measurement apparatus 1 according to the first embodiment includes a main unit 2 that performs optical measurement with respect to a biological tissue 6, which is an object to be measured, to detect characteristics of the biological tissue 6, and a probe 3 for measurement, which is inserted into a subject. The probe 3 has flexibility, a proximal end 32 thereof is detachably connected to the main unit 2, light supplied from a proximal end 32 thereof is emitted to the biological tissue 6 from the distal end 33 by the main unit 2 connected thereto, and reflected light and scattered light which are returned light from the biological tissue 6 entering from the distal end 33 are output from the proximal end 32 to the main unit 2.

The main unit 2 includes a power supply 21, a light source unit 22, a connector 23, a measurement unit 24, an input unit 25, an output unit 26, a control unit 27, and a storage unit 28.

The power supply 21 supplies electric power to each element of the main unit 2.

The light source unit 22 generates and outputs light to be emitted onto the biological tissue 6. The light source unit 22 is implemented using a light source, which is a low-coherent light source such as a white light-emitting diode (LED) emitting white light, a xenon lamp, a halogen lamp, or an LED, and one or a plurality of lenses (not illustrated). The light source unit 22 supplies low-coherent light to be emitted onto a target to the irradiation fiber 5 of the probe 3 described below.

The connector 23 is used to detachably connect the proximal end 32 of the probe 3 to the main unit 2. The connector 23 supplies, to the probe 3, light emitted from the light source unit 22 and outputs, to the measurement unit 24, the returned light output from the probe 3.

The measurement unit 24 performs spectrometry on the light output from the probe 3 and returned from the biological tissue 6. The measurement unit 24 is implemented using a plurality of spectrophotometers. The measurement unit 24 measures spectral components, an intensity, and the like of the returned light output from the probe 3 to perform measurement per wavelength. The measurement unit 24 outputs results of the measurement to the control unit 27.

The input unit 25 is implemented using a push-type switch and the like. The input unit 25 receives instruction information for instructing activation of the main unit 2 and other types of instruction information and input them to the control unit 27 by manipulation of the switch and the like.

The output unit 26 outputs information related to various types of processes in the optical measurement apparatus 1. The output unit 26 is implemented using a display, a speaker, a motor, or the like and outputs the information related to various processes in the optical measurement apparatus 1 by outputting image information, audio information, or vibration.

The control unit 27 controls processing operations of each element in the main unit 2. The control unit 27 is implemented using a central processing unit (CPU) and a semiconductor memory such as a random access memory (RAM). The control unit 27 controls operations of the main unit 2 by transferring or the like instruction information and data with respect to each element of the main unit 2. The control unit 27 causes the storage unit 28 to store the results of the measurement by the measurement unit 24. The control unit 27 has a computation unit 27a.

The computation unit 27a performs a plurality of types of computation processes based on the results of the measurement by the measurement unit 24 to compute a characteristic value related to characteristics of the biological tissue 6. A type of the characteristic value to be obtained, which is the characteristic value to be computed by the computation unit 27a, is set according to the instruction information input from the input unit 25 through manipulation by an operator.

The storage unit 28 stores an optical measurement program that causes the main unit 2 to execute the optical measurement process and various types of information related to the optical measurement process. The storage unit 28 stores each measurement result from the measurement unit 24. In addition, the storage unit 28 stores the characteristic value computed by the computation unit 27a.

The probe 3 has the proximal end 32 detachably connected to the connector 23 of the main unit 2 and the distal end 33 that makes direct contact with a biological tissue. The distal end 33 emits light supplied from the light source unit 22 and receives scattered light from an object to be measured. When the LEBS technique is used, the probe 3 is provided with a plurality of light-receiving fibers for receiving at least two scattered light beams having different scattering angles.

Specifically, the probe 3 has an irradiation fiber 5 that propagates light from the light source unit 22 supplied from the proximal end 32 and irradiates the biological tissue 6 with the light from the distal end 33, and two light-receiving fibers 7 and 8, each of which propagates the scattered light and the reflected light from the biological tissue 6 entering from the distal end 33 and outputs the light from the proximal end 32. The distal ends of the irradiation fiber 5 and the light-receiving fibers 7 and 8 are provided with a rod 34, which has transparency and is an optical member. The rod 34 is cylindrically shaped such that distances between a surface of the biological tissue 6 and the distal ends of the irradiation fiber 5 and the light-receiving fibers 7 and 8 are constant. Although the probe 3 including two light-receiving fibers 7 and 8 is described as an example in Fig. 1, the probe 3 may have three or more light-receiving fibers if at least two or more scattered light beams having different scattering angles are able to be received. In addition, the probe 3 is replaced with an unused probe per measurement. A distal end portion of the probe 3 may be configured to be removable and replaceable with an unused distal end portion per measurement. Each of these configurations is applicable to other embodiments described below.

Outside the distal end 33 of the probe 3, a balloon 41 formed of a light-shielding material is provided. The balloon 41 has, inside thereof, a space for supplying a fluid and is inflated in a radial direction of the probe when the fluid is supplied to the space. The balloon 41 is formed of an elastic material so as to be inflatable.

The probe 3 has a fluid supply tube 42 inside that delivers the fluid for inflating the balloon 41. A distal end of the fluid supply tube 42 communicates with the balloon 41, and a proximal end thereof extends from a proximal end 32 side of the probe 3. A pump 45 that sends out a fluid 44 stored in a reservoir 43 is connected to the proximal end of the fluid supply tube 42. The fluid 44 in the reservoir 43 is supplied to a space inside the balloon 41 via the fluid supply tube 42 by driving of the pump 45. The fluid 44 includes, for example, a physiological salt solution.

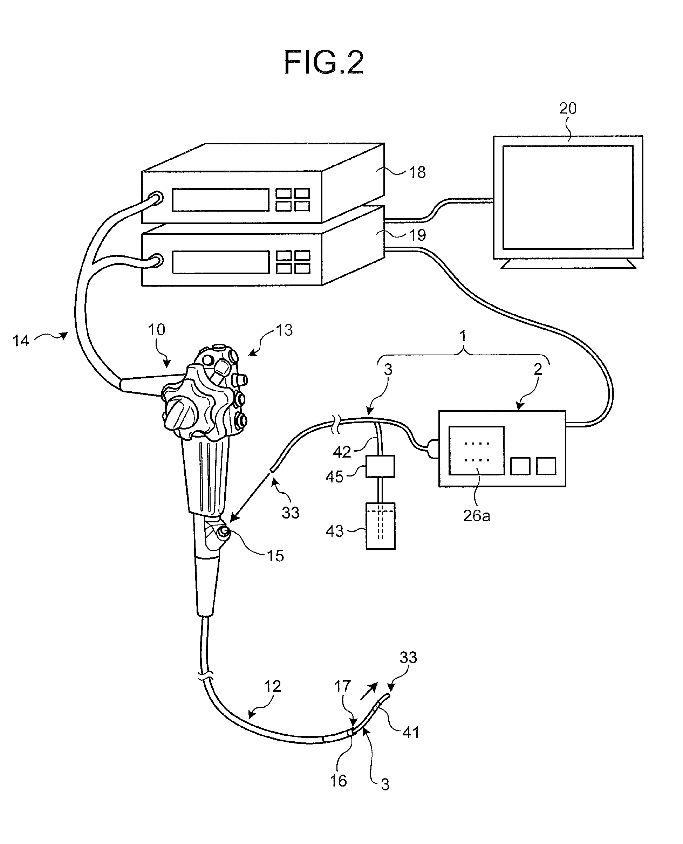

The optical measurement apparatus 1 illustrated in Fig. 1 is often combined with an endoscope system that observes internal organs such as digestive organs. Fig. 2 is a diagram illustrating a configuration of an endoscope system and a mode of installing the probe 3 in the optical measurement apparatus 1. In Fig. 2, a flexible universal cord 14 extending from a lateral portion of a manipulation unit 13 is connected to a light source device 18 and a signal processing device 19 that processes a subject image captured at a distal end portion 16 of the endoscope 10. The signal processing device 19 is connected to a display 20. The display 20 displays various types of information related to examination including the subject image processed by the signal processing device 19.

The probe 3 is inserted from a probe channel insertion hole 15 in the vicinity of the manipulation unit 13 of the out-of-body portion of the endoscope 10 inserted inside a subject as indicated by an arrow. The distal end 33 of the probe 3 passes inside an insertion portion 12 and protrudes from an aperture 17 of the distal end portion 16 connected to a probe channel as indicated by an arrow. As a result, the probe 3 is inserted into the subject and optical measurement is startable.

A predetermined surface of the main unit 2 has a display screen 26a that displays and outputs a characteristic value or the like computed by the computation unit 27a, and a switch or the like forming a part of the input unit 25. As illustrated in Fig. 2, the main unit 2 of the optical measurement apparatus 1 and the signal processing device 19 may be connected to each other, and various types of information processed in the optical measurement apparatus 1 may be output to the signal processing device 19 and displayed on the display 20.

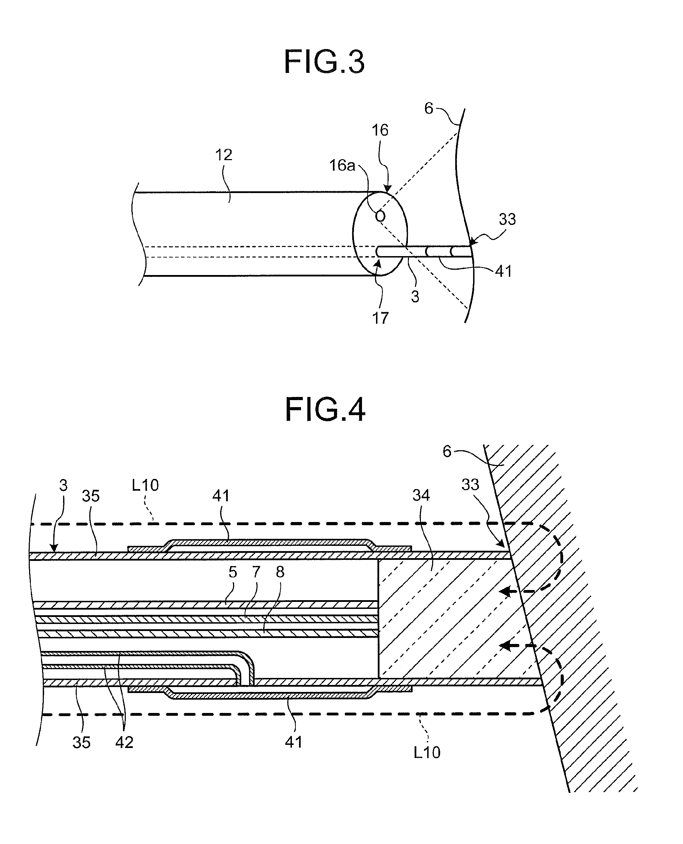

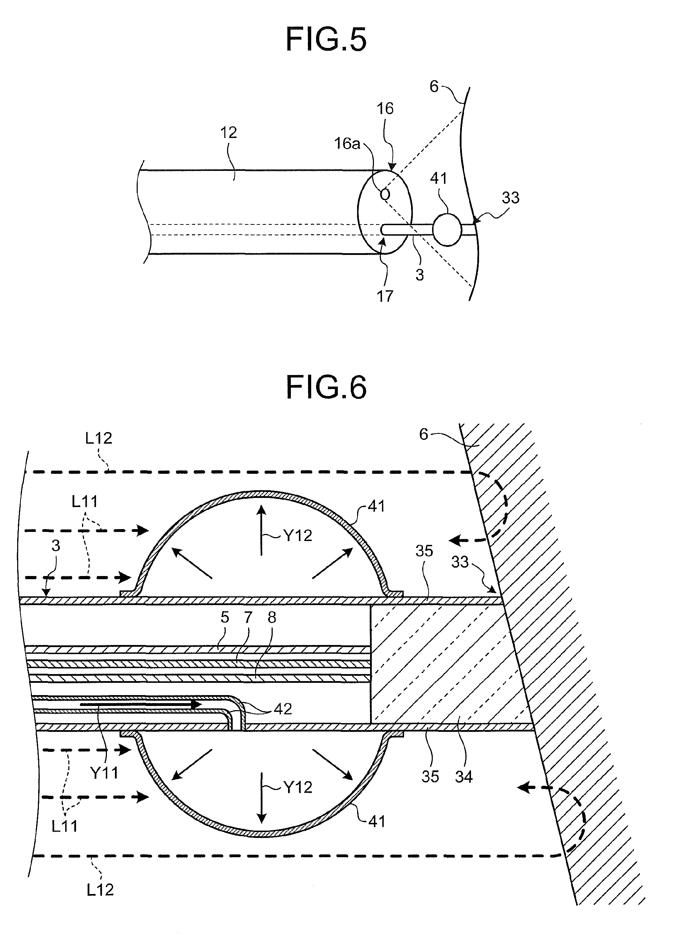

Here, a state of the probe 3 inserted into the insertion portion 12 of the endoscope 10 will be described with reference to Figs. 3 and 6. Fig. 3 is a schematic diagram illustrating a state in which the probe 3 has been inserted into the insertion portion 12 of the endoscope 10. Fig. 4 is a cross-sectional view illustrating a distal end portion of the probe 3 of Fig. 3 cut along a central axis of a longitudinal direction of the probe 3. Fig. 5 is a schematic diagram illustrating a state of the probe 3 upon measurement. Fig. 6 is a cross-sectional view illustrating the distal end portion of the probe 3 of Fig. 5 cut along the central axis of the longitudinal direction of the probe 3.

As illustrated in Figs. 3 and 4, first, in order to protrude the distal end 33 of the probe 3 smoothly from the endoscope 10, until the distal end 33 of the probe 3 has been protruded from the aperture 17 of the distal end portion 16 of the insertion portion 12 of the endoscope 10 and pushed against the biological tissue 6, the balloon 41 is maintained in a deflated state. That is, while the pump 45 is not being operated to send out and the fluid 44 is not being supplied into the fluid supply tube 42, the probe 3 is pushed into the insertion portion 12 until the distal end 33 of the probe 3 is pushed against the biological tissue 6.

Here, as illustrated in Fig. 4, a side face of the probe 3 is covered by a coating material 35 having a light-shielding ability, and a distal end face of the rod 34 is exposed to the outside. When the balloon 41 is not inflated, endoscopic illumination light L10 emitted from an illumination window 16a of a distal end of the insertion portion 12 of the endoscope 10 (see Fig. 3) may enter from a lateral side of the distal end 33 of the probe 3 into the probe 3, or enter into the probe 3 via a surface layer of the biological tissue 6 and reach the light-receiving fibers 7 and 8.

Therefore, after pushing the distal end 33 of the probe against the biological tissue 6, the pump 45 is operated to supply the fluid 44 to the fluid supply tube 42. As a result, as indicated by an arrow Y11 of Figs. 5 and 6, the fluid 44 is supplied to an internal space of the balloon 41 via the fluid supply tube 42 and the balloon 41 is inflated in a radial direction of the probe 3 as indicated by an arrow Y12. Since the balloon 41 is formed of a light-shielding material, by inflating, the balloon 41 light-shields a region including a cross section of the probe 3, the cross section being perpendicular to a long axis of the probe 3, the region having an area larger than that of the cross section.

When the balloon 41 is inflated like this, the endoscopic illumination light L11 emitted from the illumination window 16a at the distal end of the insertion portion 12 of the endoscope 10 is light-shielded by the inflated balloon 41 before it reaches the distal end 33 of the probe 3 as illustrated in Fig. 6. In addition, similarly to endoscopic illumination light L12, some light beams may reach the biological tissue 6 without being light-shielded by the balloon 41, but because a distance between the distal end 33 of the probe 3 and a position at which the endoscopic illumination light L12 reaches the biological tissue 6 increases as much as the inflation of the balloon 41, the endoscopic illumination light L12 that has reached the biological tissue 6 attenuates inside the biological tissue 6 before reaching the probe 3 and will not reach the light-receiving fibers 7 and 8.

Therefore, when the balloon 41 has been inflated, it is possible to avoid the endoscopic illumination light from reaching the light-receiving fibers 7 and 8. As a result, it is possible to obtain a measurement value having little noise caused by the endoscopic illumination light. Therefore, optical measurement of the optical measurement apparatus 1 is preferably started after the balloon 41 has been inflated. Alternatively, measurement data measured after the balloon 41 has been inflated are preferably employed for analyzing characteristics of the biological tissue 6. A distal end face of the rod 34 is cut out obliquely with respect to the longitudinal direction of the probe 3 such that unnecessary light emitted from the irradiation fiber 5 and merely reflected by the distal end face of the rod 34, before reaching the biological tissue 6, does not reach the light-receiving fibers 7 and 8. In addition, when the probe 3 is pulled out from the insertion portion 12, a pull-out process may be performed after deflating the balloon 41.

In this manner, according to the first embodiment, by inflating the balloon 41 at the distal end of the probe 3, it is possible to avoid the endoscopic illumination light from reaching the light-receiving fibers 7 and 8. Therefore, when the balloon 41 has been inflated, it is possible to obtain a measurement value having little noise caused by the endoscopic illumination light and ensure detection accuracy of characteristics of a biological tissue.

According to the first embodiment, with the balloon 41 being in the deflated state, the probe 3 is inserted into the insertion portion 12 of the endoscope 10 and pulled out from the insertion portion 12. Therefore, it is possible to smoothly perform the process of inserting the probe 3 into the insertion portion 12 and the process of pulling out the probe 3 from the insertion portion 12, without damaging an inner wall of the insertion portion 12 of the endoscope 10.

### (Second Embodiment)

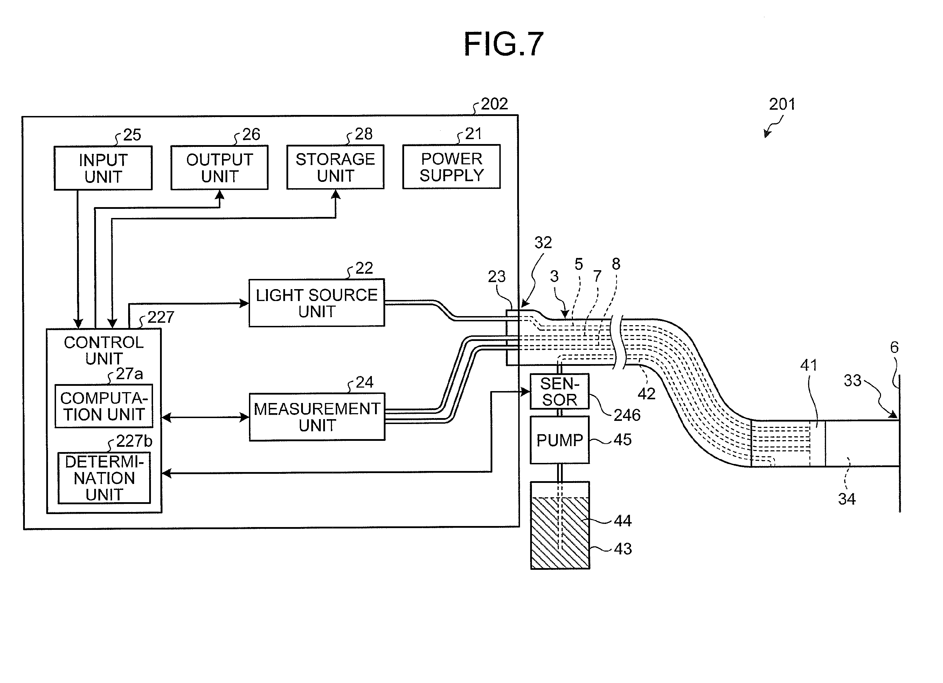

Next, a second embodiment will be described. Fig. 7 is a schematic diagram illustrating a schematic configuration of an optical measurement apparatus according to the second embodiment.

As illustrated in Fig. 7, compared to the optical measurement apparatus 1 of Fig. 1, the optical measurement apparatus 201 according to the second embodiment further includes a sensor 246 that detects a pressure in the fluid supply tube 42. In addition, the optical measurement apparatus 201 includes a main unit 202 instead of the main unit 2 of Fig. 1, the main unit 202 having a control unit 227.

As compared to the control unit 27 of Fig. 1, the control unit 227 further includes a determination unit 227b. The determination unit 227b detects an inflated state of the balloon 41 based on the pressure in the fluid supply tube 42 detected by the sensor 246, and causes a light emission process by the light source unit 22 and a spectrometry process by the measurement unit 24 to be startable when inflation of the balloon 41 is detected.

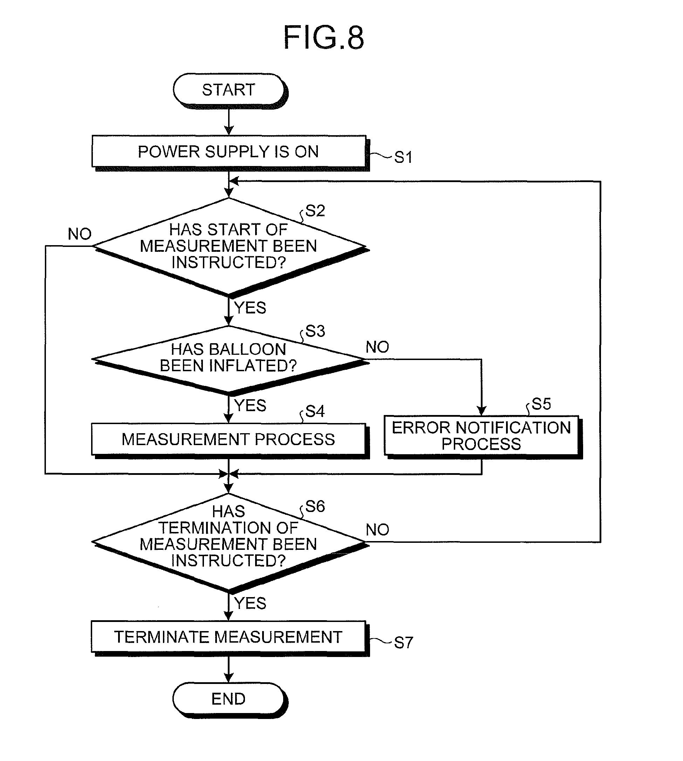

Next, a processing sequence of an optical measurement process in the optical measurement apparatus 201 of Fig. 7 will be described with reference to Fig. 8. Fig. 8 is a flowchart illustrating an optical measurement processing sequence of the optical measurement apparatus 201 of Fig. 7.

As illustrated in Fig. 8, after a power supply of the optical measurement apparatus 201 is turned on (step S1), the control unit 227 determines whether start of measurement has been instructed based on instruction information that instructs the start of measurement from the input unit 25 (step S2). If the control unit 227 determines that the start of measurement has been instructed (YES in step S2), the determination unit 227b determines whether balloon 41 has been inflated based on the pressure in the fluid supply tube 42 detected by the sensor 246 (step S3). If the determination unit 227b determines that the balloon 41 has been inflated (YES in step S3), the determination unit 227b causes the light source unit 22 to start the light emission process, the measurement unit 24 to start the spectrometry process, and the optical measurement process with respect to the biological tissue 6 to be executed (step S4).

If the determination unit 227b determines that the balloon 41 has not been inflated (NO in step S3), the output unit 26 performs an error notification process of notifying that measurement is not startable because the balloon 41 has not been inflated (step S5). In this error notification process, the determination unit 227b causes the output unit 26 to output that the measurement is not startable by audio output, display output, or both of sound output and display output.

If the control unit 227 determines that the start of measurement has not been instructed (NO in step S2), or if the measurement process of step S4 is performed, or if the error notification process of step S5 is performed, the control unit 227 determines whether termination of measurement has been instructed based on instruction information that instructs the termination of measurement from the input unit 25 (step S6). If the control unit 227 determines that the termination of measurement has been instructed (YES in step S6), the control unit 227 causes the light emission process by the light source unit 22 and the spectrometry process by the measurement unit 24 to be terminated (step S7), and ends the measurement process for the biological tissue 6. If the control unit 227 determines that the termination of measurement has not been instructed (NO in step S6), the control unit 227 returns to step S2, and determines whether the start of measurement has been instructed based on the instruction information that instructs the start of measurement from the input unit 25 (step S2).

In this manner, in the optical measurement apparatus 201 according to the second embodiment, even when the start of measurement has been instructed, the light emission process by the light source unit 22 and the spectroscopic measurement process by the measurement unit 24 are started only when the balloon 41 has been inflated. Therefore, it is possible to reliably obtain a measurement value having little noise caused by the endoscopic illumination light.

In the optical measurement apparatus 201 according to the second embodiment, the control unit 227 may control a driving process of the pump 45. In this case, if the determination unit 227b determines that the balloon 41 has not been inflated after the start of measurement had been instructed, the pump 45 may be driven to supply the fluid 44 into the balloon 41 to automatically inflate the balloon 41. Further, the determination unit 227b may detect the state of inflation of the balloon 41 based on a driving state of the pump 45 instead of or additionally to the pressure in the fluid supply tube 42 detected by the sensor 246.

In the optical measurement apparatus 201, if the determination unit 227b determines that the start of measurement is not instructed for a predetermined time or longer after the balloon 41 has been inflated, the pump 45 may be driven to suction the fluid 44 out from the inside of the balloon 41 and automatically deflate the balloon 41, to facilitate other processes including endoscopic observation.

### (Third Embodiment)

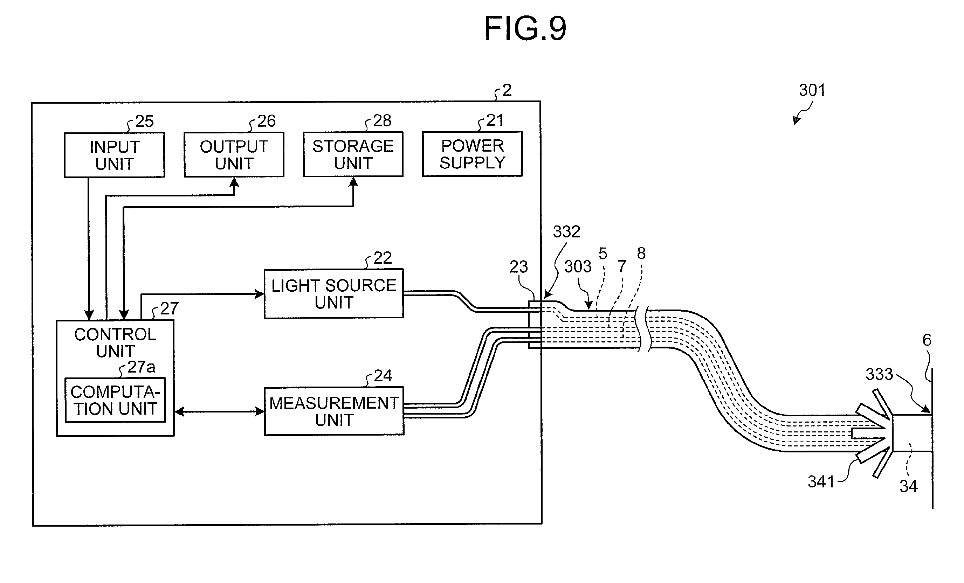

Next, a third embodiment will be described. Fig. 9 is a schematic diagram illustrating a schematic configuration of an optical measurement apparatus according to the third embodiment. As illustrated in Fig. 9, an optical measurement apparatus 301 according to the third embodiment, as compared to the optical measurement apparatus 1 of Fig. 1, has a configuration excluding the reservoir 43 and the pump 45 and includes, instead of the probe 3 of Fig. 1, a probe 303 having a configuration excluding the fluid supply tube 42 as compared to the probe 3. A proximal end 332 of the probe 303 is connected to the main unit 2 through the connector 23.

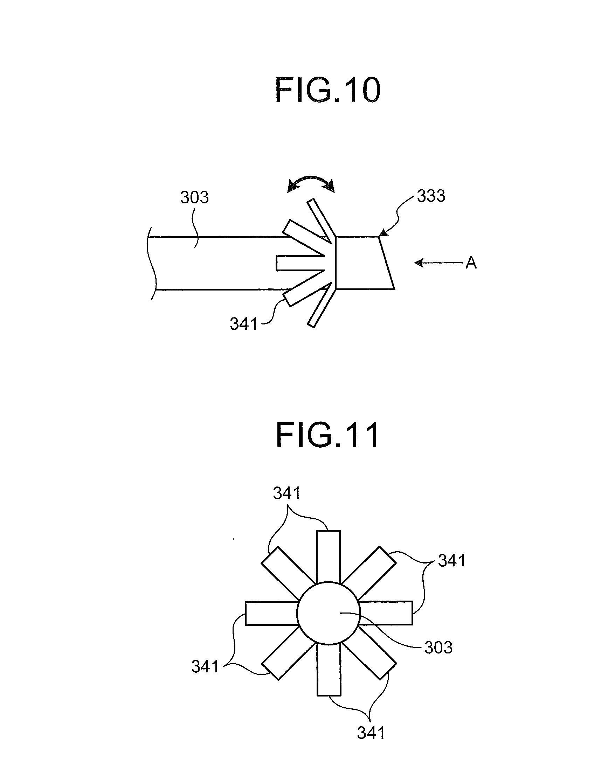

Outside a distal end 333 of the probe 303, a light-shielding plate 341 formed of a light-shielding resin material is provided. The light-shielding plate 341 is made of, for example, rubber. Fig. 10 is a perspective view illustrating a distal end portion 333 of the probe 303. Fig. 11 is view taken in a direction of an arrow A of Fig. 10. As illustrated in Figs. 10 and 11, the light-shielding plate 341 has a shape of which a plurality of cuts are provided on a side face of a cylinder from a distal end side thereof. Each segment of the light-shielding plate 341 is foldable towards the distal end and towards the proximal end of the probe 303 along an outer side face of the probe 303 as indicated by an arrow of Fig. 10. Each segment of the light-shielding plate 341 is made to have a tendency to spread out in a radial direction of the probe 303 when no external force is applied as illustrated in Fig. 10.

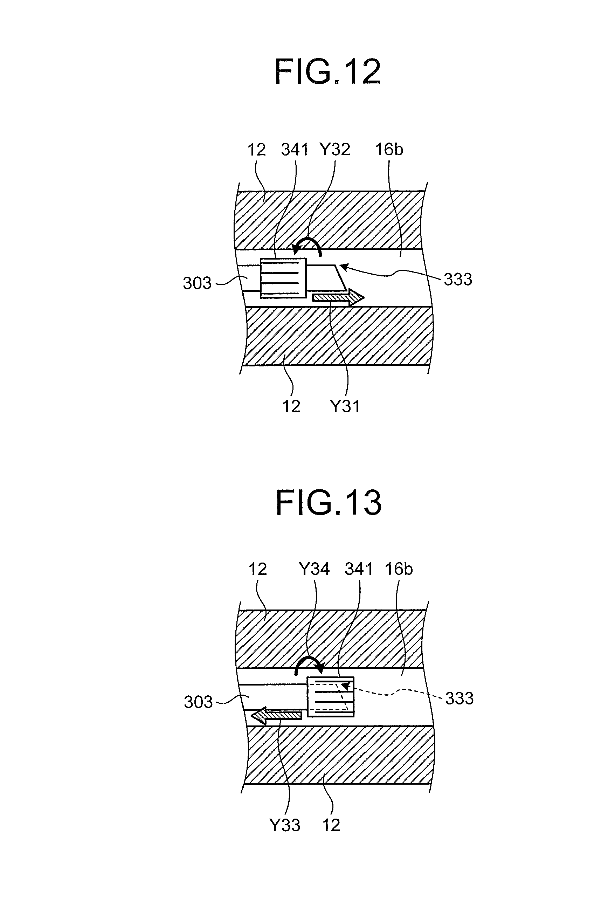

When the probe 303 is inserted into the internal channel 16b of the insertion portion 12 as indicated by an arrow Y31 of Fig. 12, it is possible to insert the probe 303 into the insertion portion 12 without damaging the inner wall of the channel 16b by folding the light-shielding plate 341 towards the proximal end of the probe 303 as indicated by an arrow Y32. Further, when the probe 303 is pulled out from the inside of the insertion portion 12 as indicated by an arrow Y33 of Fig. 13, each segment of the light-shielding plate 341 is caught by the inner wall of the channel 16b around the aperture 17 and is folded towards the distal end of the probe 303 as indicated by an arrow Y34. Therefore, it is possible to smoothly pull out the probe 303 from the inside of the insertion portion 12.

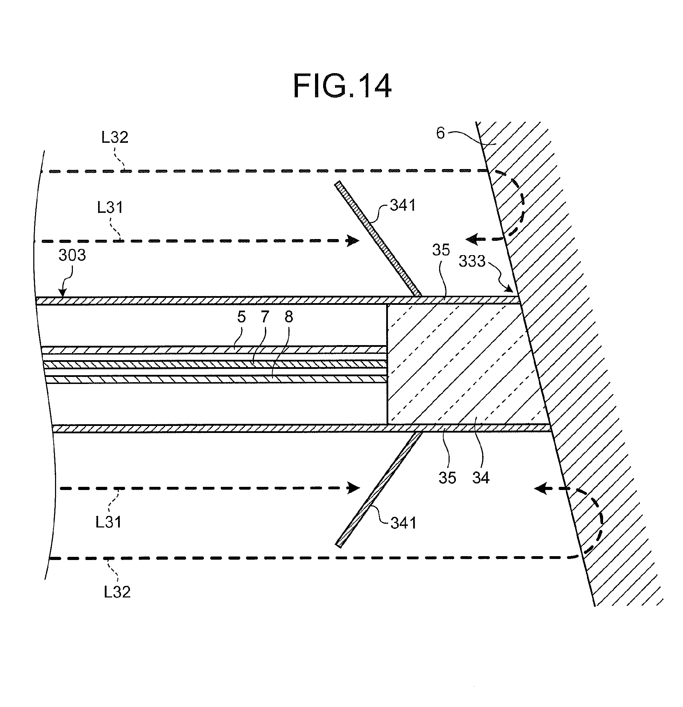

If the distal end 333 of the probe 303 protrudes from the distal end of the insertion portion 12 of the endoscope 10 for biometry, each segment of the light-shielding plate 341 naturally spreads out in the radial direction of the probe 303 as illustrated in Fig. 14 so as to light-shield a region including a cross section of the probe 303, the cross section being perpendicular to a long axis of the probe 303, the region having an area larger than that of the cross section.

In this case, as illustrated in Fig. 14, endoscopic illumination light L31 is light-shielded by each segment of the spread light-shielding plate 341 before reaching the distal end 333 of the probe 303. In addition, even when there is endoscopic illumination light L32 that has reached the biological tissue 6 without being blocked by the segments of the light-shielding plate 341, a distance between the distal end 333 of the probe 303 and a position at which the endoscopic illumination light L32 reaches the biological tissue 6 increases as much as the spread of each segment of the light-shielding plate 341. Therefore, the endoscopic illumination light L32 attenuates in the biological tissue 6 before reaching the probe 303.

Therefore, according to the third embodiment, it is possible to prevent the endoscopic illumination light from reaching the light-receiving fibers 7 and 8 using the light-shielding plate 341. Therefore, similarly to the first embodiment, it is possible to obtain a measurement value having little noise caused by the endoscopic illumination light and ensure detection accuracy for characteristics of a biological tissue 6.

### (Fourth Embodiment)

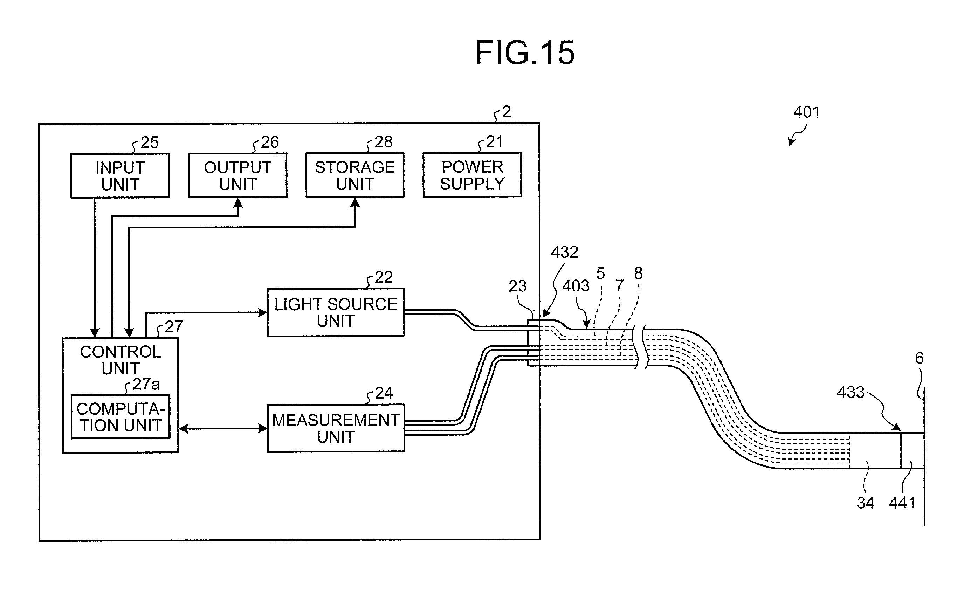

Next, a fourth embodiment will be described. Fig. 15 is a schematic diagram illustrating a schematic configuration of an optical measurement apparatus according to the fourth embodiment. As illustrated in Fig. 15, as compared to the optical measurement apparatus 301 of Fig. 9, an optical measurement apparatus 401 according to the fourth embodiment includes: instead of the probe 303, a probe 403 having a configuration excluding the light-shielding plate 341; and a hood 441 formed of a light-shielding material at a distal end 433 of the probe 403. Similarly to the probe 303, a proximal end 432 of the probe 403 is connected to the main unit 2 through the connector 23.

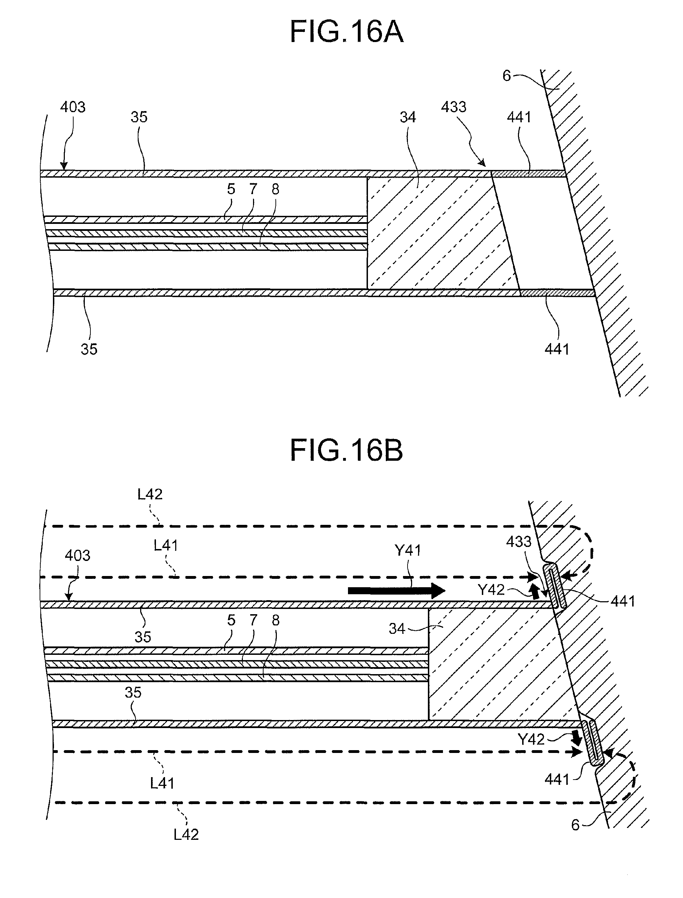

Figs. 16A and 16B are cross-sectional views illustrating a distal end portion of the probe 403 of Fig. 15 cut along a central axis of a longitudinal direction of the probe 403.

As illustrated in Fig. 16A, the hood 441 has a cylindrical shape of which a distal end thereof is open. The hood 441 is formed of an elastic resin material or the like. The hood 441 is deformed into a shape protruding outward in a radial direction of the probe 403 as indicated by an arrow Y42 when the distal end 433 of the probe 403 is pushed against the biological tissue 6 as indicated by an arrow Y41 in Fig. 16B, and light-shields a region including a cross section of the probe 403, the cross section being perpendicular to a long axis of the probe 403, the region having an area larger than that of the cross section.

In this case, even when there is endoscopic illumination light L42 that has reached without being light-shielded by a side face of the deformed hood 441, a distance between the distal end 433 of the probe 403 and a position at which the endoscopic illumination light L42 reaches the biological tissue 6 increases as much as the deformation of the side face of the hood 441. Therefore, the endoscopic illumination light L42 attenuates in the biological tissue 6 before reaching the distal end 433 of the probe 403. In addition, as illustrated in Fig. 16B, endoscopic illumination light L41 in the vicinity of an outer face of the probe 403 is light-shielded by the side face of the deformed hood 441 before reaching the distal end 433 of the probe 403.

Therefore, according to the fourth embodiment, it is possible to prevent the endoscopic illumination light from reaching the light-receiving fibers 7 and 8 using the hood 441. Accordingly, similarly to the first embodiment, it is possible to obtain a measurement value having little noise caused by the endoscopic illumination light and ensure detection accuracy for characteristics of a biological tissue 6.

### (Fifth Embodiment)

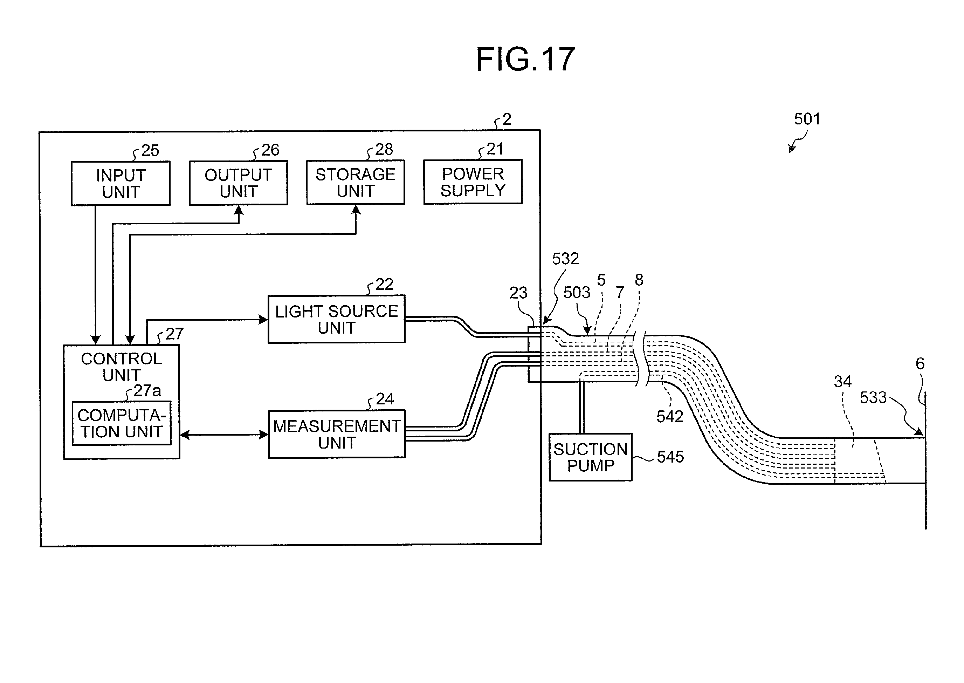

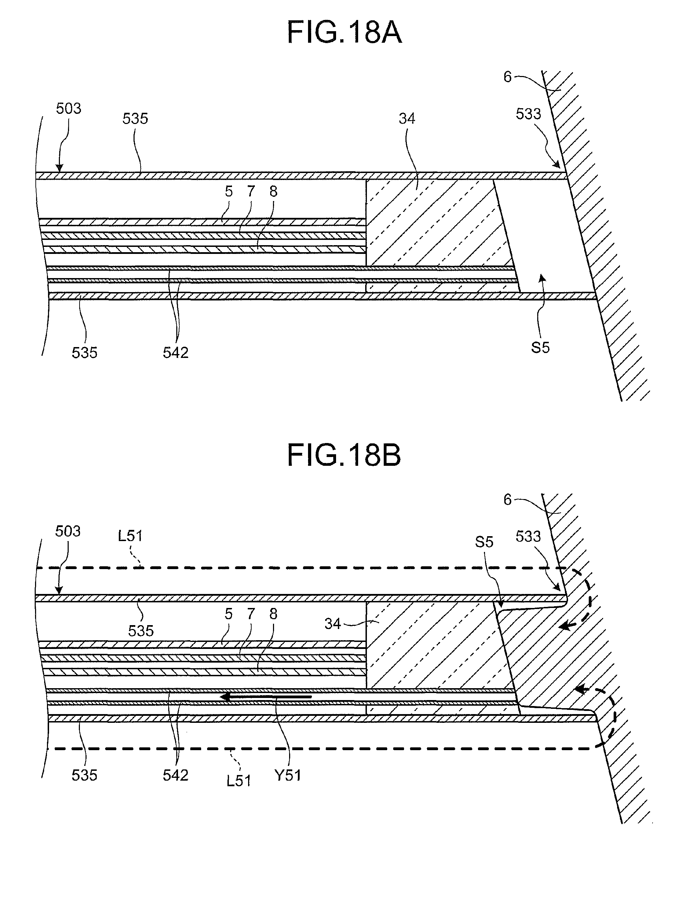

Next, a fifth embodiment will be described. Fig. 17 is a schematic diagram illustrating a schematic configuration of an optical measurement apparatus according to the fifth embodiment. Figs. 18A and 18B are cross-sectional views illustrating a distal end portion of a probe of Fig. 17 cut along a central axis in a longitudinal direction of the probe.

As illustrated in Fig. 17, an optical measurement apparatus 501 according to the fifth embodiment includes a probe 503 instead of the probe 3 of Fig. 1. Similarly to the probe 3, a proximal end 532 of the probe 503 is connected to the main unit 2 through the connector 23.

As illustrated in Figs. 17 and 18A, a side face of the probe 503 is covered by a coating material 535 having a light-shielding ability, the coating material 535 protruding distally from a distal end face of the rod 34, and the inside of a distal end 533 of the probe 503 is provided with a space S5 for draw-in. The probe 503 includes, inside thereof, a tube 542 having a distal end communicating with the space S5 and a proximal end extending from the proximal end 532 side of the probe 3. A suction pump 545 is connected to the proximal end of the tube 542.

In the optical measurement apparatus 501, as illustrated in Fig. 18A, the distal end 533 of the probe 503 is pushed against the biological tissue 6 to seal an opening at the distal end 533 of the probe 503 with the biological tissue 6. Thereafter, the suction pump 545 is driven to suction air in the tube 542 as indicated by an arrow Y51 of Fig. 18B.

As a result, pressure inside the tube 542 becomes negative, and a part of the biological tissue 6 in contact with the distal end 533 of the probe 503 is drawn into the space S5 communicating with the tube 542 and the biological tissue 6 adheres closely to the distal end of the rod 34. That is, the tube 542 and the suction pump 545 have a function of drawing in the part of the biological tissue 6 in contact with the distal end 533 of the probe 503 to the space S5 by supplying a suction pressure to the space S5.

In this case, since the biological tissue 6 is drawn into the space S5, endoscopic illumination light L51 reaching the biological tissue 6 is to go through the biological tissue 6 drawn into the space S5. That is, as compared to a case in which the biological tissue 6 is not drawn into the space S5, a distance over which the endoscopic illumination light L51 passes through the biological tissue 6 increases. Therefore, the endoscopic illumination light L51 nearly attenuates in the biological tissue 6 before reaching the light-receiving fibers 7 and 8 of the probe 503.

As described, according to the fifth embodiment, the biological tissue 6 is drawn into the space S5 at the distal end 533 of the probe 503, and the distance over which the endoscopic illumination light passes through the biological tissue 6 increases. As a result, it is possible to prevent the endoscopic illumination light from reaching the light-receiving fibers 7 and 8 and obtain a measurement value having little noise caused by the endoscopic illumination light.

### (Sixth Embodiment)

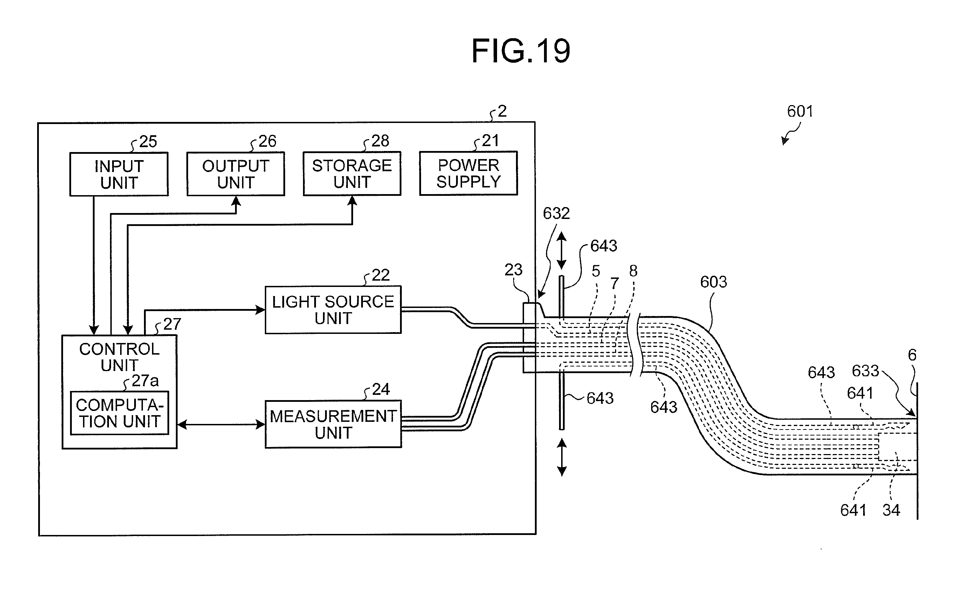

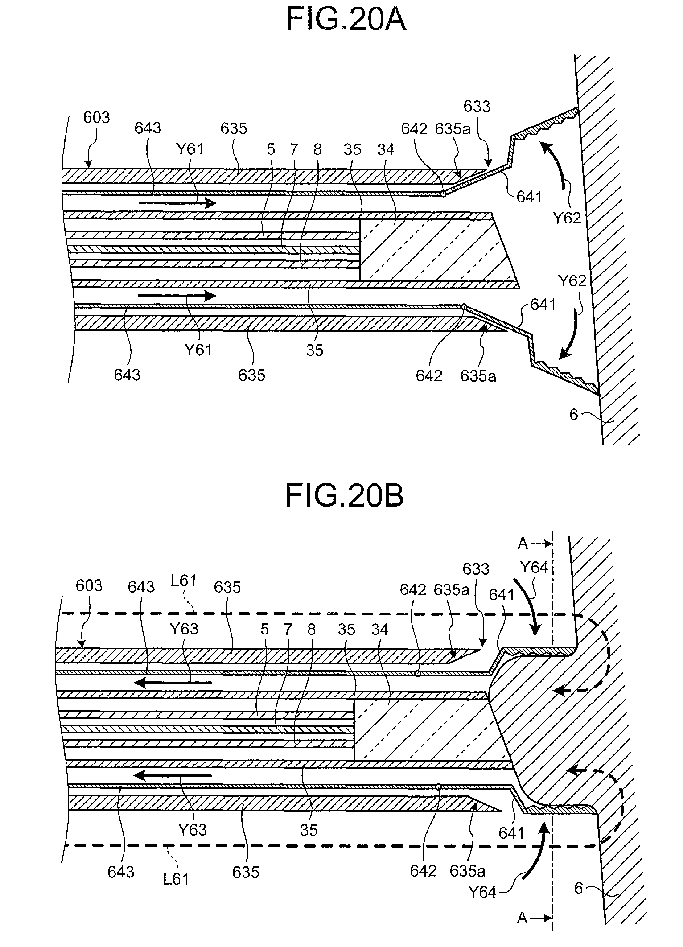

Next, a sixth embodiment will be described. Fig. 19 is a schematic diagram illustrating a schematic configuration of an optical measurement apparatus according to the sixth embodiment. Figs. 20A and 20B are cross-sectional views illustrating a distal end portion of a probe of Fig. 19 cut along a central axis of a longitudinal direction of the probe.

As illustrated in Fig. 19, an optical measurement apparatus 601 according to the sixth embodiment has a probe 603 instead of the probe 3 of Fig. 1. Similarly to the probe 3, a proximal end 632 of the probe 603 is connected to the main unit 2 through the connector 23.

As illustrated in Fig. 20A, the probe 603 has a configuration in which outside of the coating material 35, which covers the irradiation fiber 5, the light-receiving fibers 7 and 8, and the rod 34, is covered by a coating material 635. A gap is provided between the coating materials 635 and 35 from a proximal end towards a distal end, and a wire 643 is inserted slidably through the gap. A gripping claw 641 is connected to a distal end of the wire 643 via a hinge 642. The gripping claw 641 is configured to spread outward in a radial direction of the probe 603 when no external force is being applied.

A proximal end of the wire 643 extends from the proximal end 632 side of the probe 603 as illustrated in Fig. 19, and the wire 643 is able to be pushed or pulled with respect to the probe 603. The probe 603 is inserted into the insertion portion 12 of the endoscope 10 and pulled out from the insertion portion 12 in a state where the gripping claw 641 stays housed in the probe 603.

In the optical measurement apparatus 601, as illustrated in Fig. 20A, after a distal end 633 of the probe 603 is brought close to the biological tissue 6, the wire 643 is pushed in a distal end 633 direction of the probe 603 as indicated by an arrow Y61. As a result, the gripping claw 641 protrudes from the distal end 633 of the probe 603 and spreads outward in a radial direction of the probe 603 as indicated by an arrow Y62. A distal end face 635a of the coating material 635 is cut out from the inside towards the outside such that the gripping claw 641 is able to easily spread outward in the radial direction of the probe 603.

After the gripping claw 641 has protruded until it has made contact with the biological tissue 6, the wire 643 is pulled out in a proximal end 632 direction of the probe 603 and retreated towards a proximal end 632 as indicated by an arrow Y63 of Fig. 20B. As a result, the gripping claw 641, while closing inwards in a radial direction as indicated by an arrow Y64, is pulled into the inside of the coating material 635. Accordingly, a part of the biological tissue 6 making contact with the gripping claw 641 is gripped by the gripping claw 641 and pulled into the space between the gripping claws 641, so that the biological tissue 6 adheres closely to the distal end of the rod 34.

In this case, since the biological tissue 6 has been pulled into the space between the gripping claws 641, endoscopic illumination light L61 reaching the biological tissue 6 goes through the biological tissue 6 that has been pulled into the space between the gripping claws 641. That is, as compared to a case in which the biological tissue 6 has not been pulled into the space between the gripping claws 641, a distance over which the endoscopic illumination light L61 passes through the biological tissue 6 increases. Therefore, the endoscopic illumination light L61 nearly attenuates in the biological tissue 6 before reaching the light-receiving fibers 7 and 8 of the probe 603.

As described, according to the sixth embodiment, similarly to the fifth embodiment, since the distance over which the endoscopic illumination light passes through the biological tissue 6 increases, it is possible to prevent the endoscopic illumination light from reaching the light-receiving fibers 7 and 8 and obtain a measurement value having little noise caused by the endoscopic illumination light.

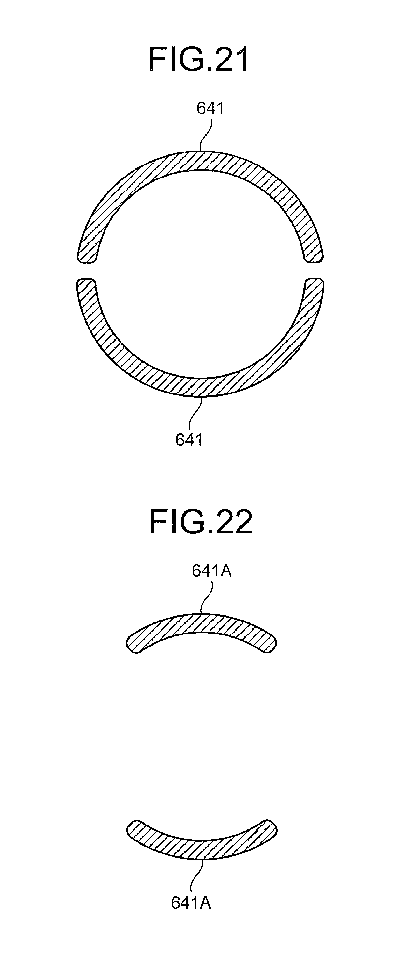

Fig. 21 is a cross-sectional view taken along a line A-A of Fig. 20B. Fig. 22 is another example of the cross-sectional view taken along the line A-A of Fig. 20B. The gripping claw 641 may be configured such that a cross section of a portion that grips the biological tissue 6 is semicircular, and the gripping claw 641 is able to grip the biological tissue 6 so that the biological tissue 6 is not exposed from a side face of the gripping claw 641, as illustrated in Fig. 21. In addition, if the distance over which the endoscopic illumination light passes through the biological tissue 6 is sufficiently ensurable, a cross section of the portion that grips the biological tissue may have an arc shape of which a semicircle is further cut out, as illustrated by the gripping claw 641A of Fig. 22.

### Reference Signs List

- 1: OPTICAL MEASUREMENT APPARATUS
- 2, 202: MAIN UNIT
- 3, 303, 403, 503, 603: PROBE
- 5: IRRADIATION FIBER
- 6: BIOLOGICAL TISSUE
- 7, 8: LIGHT RECEIVING FIBER
- 10: ENDOSCOPE
- 12: INSERTION PORTION
- 13: MANIPULATION UNIT
- 14: UNIVERSAL CORD
- 15: PROBE CHANNEL INSERTION HOLE
- 16: DISTAL END PORTION
- 17: APERTURE
- 18: LIGHT SOURCE DEVICE
- 19: SIGNAL PROCESSING DEVICE
- 29: DISPLAY
- 21: POWER SUPPLY
- 22: LIGHT SOURCE UNIT
- 23: CONNECTOR
- 24: MEASUREMENT UNIT
- 25: INPUT UNIT
- 26: OUTPUT UNIT
- 27, 227: CONTROL UNIT
- 27a: COMPUTATION UNIT
- 28: STORAGE UNIT
- 34: ROD
- 35, 535, 635: COATING MATERIAL
- 41: BALLOON
- 42: FLUID SUPPLY TUBE
- 43: RESERVOIR
- 44: FLUID
- 45: PUMP
- 227b: DETERMINATION UNIT
- 246: SENSOR
- 341: LIGHT-SHIELDING PLATE
- 441: HOOD
- 542: TUBE
- 545: SUCTION PUMP
- 641, 641A: GRIPPING CLAW
- 642: HINGE
- 643: WIRE

## Claims

1. An optical measurement apparatus that performs spectrometry on returned light reflected or scattered by a biological tissue and obtains a characteristic value of the biological tissue, the optical measurement apparatus comprising:
a probe having: an irradiation fiber that propagates light supplied from a proximal end thereof and emits the light from a distal end thereof; and a plurality of light-receiving fibers, each of which propagates light entering from a distal end thereof and outputs the light from a proximal end thereof; and
a light-shielding member provided at a distal end of the probe to light-shield a region including a cross section of the probe, the cross section being perpendicular to a long axis of the probe, the region having an area larger than that of the cross section.

2. The optical measurement apparatus according to claim 1, wherein
the light-shielding member is a balloon that inflates in a radial direction of the probe,
the probe has a fluid supply tube of which a distal end communicates with inside of the balloon and a proximal end extends from a proximal end portion of the probe, and
the optical measurement apparatus further comprises a fluid supply unit that supplies a fluid to the fluid supply tube.

3. The optical measurement apparatus according to claim 2, further comprising:
a light source that generates light to emit the biological tissue and supplies the light to the irradiation fiber;
a measurement unit that performs spectrometry on the returned light from the biological tissue output by each of the plurality of the light-receiving fibers;
a detection unit that detects a state of inflation of the balloon; and
a control unit that causes a light emission process by the light source and a spectrometry process by the measurement unit to be startable when inflation of the balloon is detected by the detection unit.

4. The optical measurement apparatus according to claim 1, wherein the light-shielding member is a light-shielding plate that is foldable along an outer side face of the probe.

5. The optical measurement apparatus according to claim 1, wherein the light-shielding member is a hood that deforms into a shape protruding towards a radial direction of the probe when the distal end of the probe is pushed against the biological tissue.

6. An optical measurement apparatus comprising a probe having an irradiation fiber that propagates light supplied from a proximal end thereof and emits the light from a distal end thereof and a plurality of light-receiving fibers, each of which propagates light entering from a distal end thereof and outputs the light from a proximal end thereof, the optical measurement apparatus performing spectrometry on returned light reflected or scattered by a biological tissue and obtains a characteristic value of the biological tissue, wherein the optical measurement apparatus further comprises:
a draw-in unit that causes ambient light coming from outside of the probe to attenuate within the biological tissue before the ambient light reaches the light-receiving fibers by drawing the biological tissue making contact with the distal end of the probe into a space for draw-in provided in the probe.

7. The optical measurement apparatus according to claim 6, wherein the draw-in unit is a suction pressure supply unit that supplies a suction pressure to the space.

8. The optical measurement apparatus according to claim 6, wherein the draw-in unit is a gripping unit that grips a part of the biological tissue and pulls the part of the biological tissue into the space.

9. A probe having: an irradiation fiber that propagates light supplied from a proximal end thereof and emits the light from a distal end thereof; and a plurality of light-receiving fibers, each of which propagates light entering from a distal end thereof and outputs the light from a proximal end thereof; wherein the probe comprises:
a light-shielding member provided at a distal end of the probe to light-shield a region including a cross section of the probe, the cross section being perpendicular to a long axis of the probe, the region having an area larger than that of the cross section.

10. The probe according to claim 9, wherein the light-shielding member is a balloon that inflates in a radial direction of the probe, and the probe further comprises a fluid supply tube, of which a distal end communicates with inside of the balloon and a proximal end extends from a proximal end portion of the probe.

11. The probe according to claim 9, wherein the light-shielding member is a light-shielding plate that is foldable along an outer side face of the probe.

12. The probe according to claim 9, wherein the light-shielding member is a hood that deforms into a shape protruding towards a radial direction of the probe when the distal end of the probe is pushed against the biological tissue.
